# EUROPEAN PATENT APPLICATION

(11) **EP 4 506 012 A1**
(43) Date of publication of application: **12.02.2025**
(21) Application number: 22934307.4
(22) Date of filing: 01.04.2022
(51) Int. Cl.: A61K 35/36, A61P 25/28

(54) **USE OF EXTRACTS FROM RABBIT SKIN INFLAMED BY VACCINIA VIRUS IN TREATMENT OF ALZHEIMER'S DISEASE**

(71) Applicant: Zodiac Bio-Tech Limited, Kowloon Hong Kong (HK)
(72) Inventor: LAU, Shing Hing, Hong Kong (CN)
(74) Representative: Rüger Abel Patentanwälte PartGmbB
(86) International application number: PCT/CN2022/084779
(87) International publication number: WO 2023/184470

(57) **Abstract**

The present invention relates to the therapeutic use of extract from rabbit skin inflamed by vaccinia virus. More specifically, the present invention relates to the use of extract from rabbit skin inflamed by vaccinia virus for treating Alzheimer's disease. **In** addition, the present invention relates to the use of extract from rabbit skin inflamed by vaccinia virus for protecting neurological function of the brain or alleviating damage to neurological function of the brain in a patient suffering from Alzheimer's disease. **In** addition, the extract from rabbit skin inflamed by vaccinia virus can be Lepalvir.

## Description

### Field of invention

The invention relates to the field of medicine. Specifically, the present invention relates to new therapeutic use of extract from rabbit skin inflamed by vaccinia virus. More specifically, the present invention relates to a medical use of extract from rabbit skin inflamed by vaccinia virus for treating Alzheimer's disease.

### Background Art

Alzheimer's disease (AD) is a disease that progresses slowly and worsens over time, and is directly caused by the gradual degeneration of neurons. The most common early symptom is loss of short-term memory. As the disease progresses, symptoms may gradually appear, including language disorders, disorientation, emotional instability, loss of motivation, lack of self-care, and many behavioral problems. When the condition worsens, patients often become disconnected from their family or society, and gradually lose physical function, eventually leading to death.

In AD, hippocampus is the first area to be damaged, with symptoms such as memory decline and loss of sense of direction (see Yangling Mu et al., Adult hippocampal neurogenesis and its role in Alzheimer's disease, Mol Neurodegener, 2011; 6: 85). The progression of AD is associated with the deposition of β-amyloid (Aβ) plaques and Tau protein in the brain (see Clive Ballard et al., Alzheimer's disease, The Lancet. 2011 March, 377 (9770): 1019-1031). Recent studies have found that neuroinflammation or microglial activation is involved in the spread of tau protein tangles in the neocortex in Alzheimer's disease, which in turn leads to the occurrence of cognitive dysfunction in a patient suffering from Alzheimer's disease (see Tharick A. Pascoal et al., Microglial activation and tau propagate jointly across Braak stages, Nature Medicine, 27, pages 1592-1599 (2021 August)). Microglial activation is part of the human brain's immune response and is a key factor associated with the development of Alzheimer's disease. Excessive activation of microglia is not just an inflammatory epiphenomenon, but also a key upstream mechanism that is crucial to the development of AD. Studies have also found that neuroinflammation and certain inflammatory cytokines are involved in the pathology of AD (see Rishika Dhapola et al., Recent advances in molecular pathways and therapeutic implications targeting neuroinflammation for Alzheimer's disease, Inflammopharmacology volume 29, pages 1669-1681 (2021 November)). Therefore, reducing Aβ plaque deposition, alleviating neuroinflammation, and inhibiting microglial activation can be considered as effective means to treat or alleviate AD.

There is a need in the art for drugs that are effective in treating Alzheimer's disease.

The extract from rabbit skin inflamed by vaccinia virus refers to active substances extracted from the rabbit skin that has been inflamed after being vaccinated with vaccinia virus. This extract from rabbit skin inflamed by vaccinia virus is commercially available under the trade name Lepalvir for pain treatment. The extract has been shown to be clinically safe and has no obvious side effects. In addition, WO2020/211009 discloses the use of extract from rabbit skin inflamed by vaccinia virus for the treatment of hematopoietic system damage; and WO 2020/248240 discloses the use of extract from rabbit skin inflamed by vaccinia virus for the treatment of cancer, and their entire contents are incorporated herein by reference.

### Summary of the invention

The purpose of the present invention includes providing a drug for preventing, alleviating or treating Alzheimer's disease. The technical problem of the present invention is solved by providing extract from rabbit skin inflamed by vaccinia virus, preferably Lepalvir.

In general, the inventors found that extract from rabbit skin inflamed by vaccinia virus can effectively prevent, treat or alleviate Alzheimer's disease.

In one aspect, the present invention relates to use of extract from rabbit skin inflamed by vaccinia virus in the preparation of a medicament for preventing or treating Alzheimer's disease in a patient. In one aspect, the present invention relates to extract from rabbit skin inflamed by vaccinia virus for use in preventing or treating Alzheimer's disease in a patient. In one aspect, the present invention relates to a method for preventing or treating Alzheimer's disease in a patient in need thereof, the method comprising administering a therapeutically effective amount of extract from rabbit skin inflamed by vaccinia virus to the patient.

In one aspect, the extract from rabbit skin inflamed by vaccinia virus or the medicament is used to protect neurological function of the brain or alleviate damage to neurological function of the brain in a patient suffering from Alzheimer's disease. Damage to neurological function of the brain can be caused by β-amyloid protein (Aβ) plaque deposition, inflammatory response of glial cells, and/or increased expression or level of inflammatory factors in the brain.

In one aspect, prevention or treatment of Alzheimer's disease is achieved by alleviating or reducing β-amyloid plaque deposition, inflammatory response of glial cells, or the expression or level of inflammatory factors in the brain.

In one aspect, the present invention relates to use of extract from rabbit skin inflamed by vaccinia virus in the preparation of a medicament for protecting neurological function of the brain or alleviating damage to neurological function of the brain in a patient suffering from Alzheimer's disease. In one aspect, the present invention relates to extract from rabbit skin inflamed by vaccinia virus for use in protecting neurological function of the brain or alleviating damage to neurological function of the brain in a patient suffering from Alzheimer's disease. In one aspect, the present invention relates to a method for protecting neurological function of the brain or alleviating damage to neurological function of the brain in a patient suffering from Alzheimer's disease, the method comprising administering a therapeutically effective amount of extract from rabbit skin inflamed by vaccinia virus to the patient.

In one aspect, protecting neurological function of the brain or alleviating damage to neurological function of the brain in a patient suffering from Alzheimer's disease is achieved by alleviating or reducing β-amyloid plaque deposition, inflammatory response of glial cells, or the expression or level of inflammatory factors in the brain. In one aspect, the present invention relates to use of extract from rabbit skin inflamed by vaccinia virus in the preparation of a medicament for alleviating or reducing β-amyloid plaque deposition in the brain of a patient suffering from Alzheimer's disease. In one aspect, the present invention relates to extract from rabbit skin inflamed by vaccinia virus for use in alleviating or reducing β-amyloid plaque deposition in the brain of a patient suffering from Alzheimer's disease. In one aspect, the present invention relates to a method for alleviating or reducing β-amyloid plaque deposition in the brain of a patient suffering from Alzheimer's disease, the method comprising administering a therapeutically effective amount of extract from rabbit skin inflamed by vaccinia virus to the patient.

In one aspect, β-amyloid plaque deposition can occur in the hippocampus or cortex of the brain.

In one aspect, the present invention relates to use of extract from rabbit skin inflamed by vaccinia virus in the preparation of a medicament for alleviating or reducing inflammatory response of glial cells in the brain of a patient suffering from Alzheimer's disease. In one aspect, the present invention relates to extract from rabbit skin inflamed by vaccinia virus for use in alleviating or reducing inflammatory response of glial cells in the brain of a patient suffering from Alzheimer's disease. In one aspect, the present invention relates to a method for alleviating or reducing inflammatory response of glial cells in the brain of a patient suffering from Alzheimer's disease, the method comprising administering a therapeutically effective amount of extract from rabbit skin inflamed by vaccinia virus to the patient.

Glial cells may also be referred to as neuroglial cells, which may be selected from astrocytes or microglia, preferably microglia. Glial cells may be cortical glial cells or hippocampal glial cells in the brain. The extract of the present invention may reduce or alleviate abnormalities or activation of cortical glial cells or hippocampal glial cells in the brain, thereby achieving prevention or treatment. Glial inflammatory response may be associated with or caused by abnormalities or activation of glial cells.

In one aspect, the present invention relates to use of extract from rabbit skin inflamed by vaccinia virus in the preparation of a medicament for alleviating or reducing the expression or level of inflammatory factors in the brain of a patient suffering from Alzheimer's disease. In one aspect, the present invention relates to extract from rabbit skin inflamed by vaccinia virus for use in alleviating or reducing the expression or level of inflammatory factors in the brain of a patient suffering from Alzheimer's disease. In one aspect, the present invention relates to a method for alleviating or reducing the expression or level of inflammatory factors in the brain of a patient suffering from Alzheimer's disease, the method comprising administering a therapeutically effective amount of extract from rabbit skin inflamed by vaccinia virus to the patient.

The inflammatory factor mainly refers to inflammatory cytokine or proinflammatory cytokine, which can be selected from interleukin-1β (IL-1β), interleukin-6 (IL-6) or tumor necrosis factor-α (TNF-α). Inflammatory factors can be inflammatory factors in the cortex or hippocampus of the brain. Therefore, the extract of the present invention can be used in a method for alleviating or reducing the expression or level of inflammatory factors in the cortex or hippocampus of a patient suffering from Alzheimer's disease.

In one aspect, the present invention relates to use of extract from rabbit skin inflamed by vaccinia virus in the preparation of a medicament for improving, enhancing or restoring cognitive functions or cognitive abilities in a patient suffering from Alzheimer's disease. In one aspect, the present invention relates to extract from rabbit skin inflamed by vaccinia virus for use in improving, enhancing or restoring cognitive functions or cognitive abilities in a patient suffering from Alzheimer's disease. In one aspect, the present invention relates to a method for improving, enhancing or restoring cognitive functions or cognitive abilities in a patient suffering from Alzheimer's disease, the method comprising administering a therapeutically effective amount of extract from rabbit skin inflamed by vaccinia virus to the patient. Cognitive functions or cognitive abilities may include perception, logical thinking, memory, language, learning, emotion control, social skills or attention.

In one aspect, the extract from rabbit skin inflamed by vaccinia virus can be used as the only active ingredient for the treatment of Alzheimer's disease. In this aspect, the extract from rabbit skin inflamed by vaccinia virus can be used as the only active ingredient for the preparation of a medicament, which is used for the treatment of Alzheimer's disease, or for protecting neurological function of the brain or alleviating damage to neurological function of the brain in a patient suffering from Alzheimer's disease, or for alleviating or reducing β-amyloid plaque deposition in the brain of a patient suffering from Alzheimer's disease, or for alleviating or reducing inflammatory response of glial cells in the brain of a patient suffering from Alzheimer's disease, or for alleviating or reducing the expression or level of inflammatory factors in the brain of a patient suffering from Alzheimer's disease. In the treatment method of the present invention, the extract from rabbit skin inflamed by vaccinia virus can be administered to the patient as the only active ingredient. "The only active ingredient" means that the extract from rabbit skin inflamed by vaccinia virus is not used, administered to a patient, or prepared as a medicament, in combination with other therapeutic agents for Alzheimer's disease. In one aspect, the present invention relates to a pharmaceutical composition comprising extract from rabbit skin inflamed by vaccinia virus and optionally a pharmaceutically acceptable carrier, adjuvant or excipient. In one aspect of the present invention, the pharmaceutically acceptable carrier, adjuvant or excipient are those that formulate the drug into an oral formulation or an injection. In one aspect, the extract from rabbit skin inflamed by vaccinia virus is formulated into an oral formulation or an injection, preferably an intramuscular injection or an intravenous injection. In one aspect, the extract from rabbit skin inflamed by vaccinia virus is Lepalvir. Accordingly, the present invention also relates to the pharmaceutical composition for the treatment of Alzheimer's disease, or for protecting neurological function of the brain or alleviate damage to neurological function of the brain in a patient suffering from Alzheimer's disease, or for alleviating or reducing β-amyloid plaque deposition in the brain of a patient suffering from Alzheimer's disease, or for alleviating or reducing the inflammatory response of glial cells in the brain of a patient suffering from Alzheimer's disease, or for alleviating or reducing the expression or level of inflammatory factors in the brain of a patient suffering from Alzheimer's disease. The present invention also relates to use of the extract from rabbit skin inflamed by vaccinia virus in the preparation of the pharmaceutical composition.

In one aspect, the extract from rabbit skin inflamed by vaccinia virus is Lepalvir.

In one aspect, the extract from rabbit skin inflamed by vaccinia virus is formulated into an oral preparation or an injection, preferably an intramuscular injection or an intravenous injection.

In one aspect, the patient is a mammal, preferably a human.

In one aspect, the extract from rabbit skin inflamed by vaccinia virus is administered to a patient, such as a human, in an amount of 0.05 U/kg to 50 U/kg, preferably 0.1 U/kg to 10 U/kg, more preferably 0.5 U/kg to 5 U/kg.

In one aspect, the prepared medicament comprises 3 U to 3000 U, preferably 6 U to 600 U, more preferably 30 U to 300 U of the extract from rabbit skin inflamed by vaccinia virus.

In one aspect, the extract from rabbit skin inflamed by vaccinia virus (preferably Lepalvir) is administered to a patient, preferably a human, in an amount of 0.05 U/kg to 50 U/kg, preferably 0.1 U/kg to 10 U/kg, more preferably 0.5 U/kg to 5 U/kg. For example, the extract from rabbit skin inflamed by vaccinia virus is administered to a patient, preferably a human, in an amount selected from the group consisting of: 0.05 U/kg, 0.06 U/kg, 0.07 U/kg, 0.08 U/kg, 0.09 U/kg, 0.1 U/kg, 0.2 U/kg, 0.3 U/kg, 0.4 U/kg, 0.5 U/kg, 0.6 U/kg, 0.7 U/kg, 0.8 U/kg, 0.9 U/kg, 1 U/kg, 1.5 U/kg, 2 U/kg, 2.5 U/kg, 3 U/kg, 3.1 U/kg, 3.2 U/kg, 3.3 U/kg, 3.4 U/kg, 3.5 U/kg, 3.6 U/kg, 3.7 U/kg, 3.8 U/kg, 3.9 U/kg, 4 U/kg, 4.5 U/kg, 5 U/kg, 5.5 U/kg, 6 U/kg, 6.5 U/kg, 7 U/kg, 7.5 U/kg, 8 U/kg, 8.5 U/kg, 9 U/kg, 9.5 U/kg, 10 U/kg, 11 U/kg, 12 U/kg, 13 U/kg, 14 U/kg, 15 U/kg, 16 U/kg, 17 U/kg, 18 U/kg, 19 U/kg, 20 U/kg, 25 U/kg, 30 U/kg, 35 U/kg, 40 U/kg, 45 U/kg, 50 U/kg and the ranges bounded by these numbers. It is known to those skilled in the art that for the dosage, human dose (U/kg or mg/kg) = mouse dose (U/kg or mg/kg) / 12.3; or human dose (U/kg or mg/kg) = mouse dose (U/kg or mg/kg) × 0.08. The above dosage can be an effective amount for treating the above-mentioned diseases in a patient. In one aspect, the extract from rabbit skin inflamed by vaccinia virus is administered by injection at the above-mentioned dose, such as intramuscular injection or intravenous injection.

In one aspect of the present invention, the prepared medicament or pharmaceutical composition comprises 3 U to 3000 U, preferably 6 U to 600 U, more preferably 30 U to 300 U of the extract from rabbit skin inflamed by vaccinia virus. The medicament or pharmaceutical composition is used to administer to a human, such as an adult. The average weight of an adult is, for example, 60 kg. Accordingly, the amount of extract from rabbit skin inflamed by vaccinia virus contained in the medicament prepared by the present invention is, for example, 3 U, 4 U, 5 U, 6 U, 7 U, 8 U, 9 U, 10 U, 15 U, 20 U, 25 U, 30 U, 35 U, 40 U, 42 U, 44 U, 45 U, 46 U, 47 U, 48 U, 49 U, 50 U, 55 U, 60 U, 65 U, 70 U, 80 U, 90 U, 100 U, 120 U, 150 U, 160 U, 170 U, 180 U, 190 U, 192 U, 194 U, 195 U, 196 U, 198 U, 200 U, 220 U, 240 U, 260 U, 280 U, 300 U, 350 U, 400 U, 500 U, 600 U, 700 U, 800 U, 900 U, 1000 U, 1500 U, 2000 U, 2500 U, 3000 U and the ranges bounded by these numbers. In one aspect, the medicament or pharmaceutical composition is prepared as an injection, such as an intramuscular injection or an intravenous injection. In one aspect, the medicament or injection is a fixed dose that cannot be divided. In one aspect, the medicament or injection cannot be divided into smaller doses within 1, 2, 3, 4, 5, 6 or 7 days. In one aspect, the medicament or injection is administered only once within 1, 2, 3, 4, 5, 6 or 7 days.

In one aspect, the extract from rabbit skin inflamed by vaccinia virus is administered to a patient every 6-72 hours, preferably 12-48 hours, more preferably 24-36 hours, more preferably 24 hours.

In one aspect, the regimen of administration to the patient is three times a day, twice a day, once a day, once every two days, once every three days, once every four days, once every five days, once every six days, once a week, once every two weeks, once every three weeks, once a month. For example, the extract of the present invention can be administered to the patient once a day.

In one aspect, the extract of the present invention is administered to the patient for at least 24 months, at least 12 months, at least 6 months, at least 2 months, at least 1 month, at least 3 weeks, at least 2 weeks, at least 10 days, at least 7 days, at least 5 days, at least 2 days, or at least 1 day.

As used herein, "extract from rabbit skin inflamed by vaccinia virus" refers to an extract, which contains active substances and is extracted from the inflamed rabbit skin inoculated with vaccinia virus using such processes as leaching, purification, and refining. This extract is usually a yellow or light yellow liquid, but it can also be dried into a solid. The injection of this extract from rabbit skin inflamed by vaccinia virus is commercially available under the trade name Lepalvir. The extract from rabbit skin inflamed by vaccinia virus of the present invention may contain peptides. For example, it has been found that the extract may contain naturally occurring peptides (i.e., not additionally added peptides). The peptides may be short peptides. Therefore, in this aspect, the extract from rabbit skin inflamed by vaccinia virus of the present invention may not be a non-proteinaceous extract.

In the preparation of the extract of the present invention, the preparation method comprises extracting rabbit skin fragments inflamed after vaccinia virus inoculation with a phenol aqueous solution, wherein preferably a phenol aqueous solution with a phenol concentration of about 1%-10%, preferably about 2%-5%, more preferably about 2% or about 3% is used at a temperature below about 12°C, such as about 0-10°C, preferably about 2-8°C, more preferably about 3-6°C, and more preferably about 4°C. In addition, the preparation method further comprises adsorbing the extract treated with the phenol aqueous solution with an adsorbent (such as activated carbon), and desorbing under alkaline conditions, wherein preferably the adsorption is carried out under acidic conditions (such as about pH 3-6, more preferably about pH 4-5, more preferably about pH 4.5), and the alkaline conditions for desorption are about pH 9-12, preferably about pH 10 or pH 11.

In one aspect, the extract from rabbit skin inflamed by vaccinia virus or Lepalvir can be prepared by a method including the following steps:
(1) The inflamed rabbit skin after inoculation with vaccinia virus is collected, fragmented, and extracted with an extraction solvent to obtain solution A;
(2) Solution A is treated with acid and heat to obtain solution B;
(3) Solution B is treated with alkali and heat to obtain solution C;
(4) Solution C is subject to adsorption and filtration under acidic conditions, and desorption under alkaline conditions to obtain solution D;
(5) Solution D is neutralized and heated to obtain solution E;
(6) Solution E is concentrated to obtain the extract; and
(7) Optionally the extract is mixed with pharmaceutically acceptable carrier, adjuvant or excipient.

In one aspect, in step (1), the rabbit is inoculated with vaccinia virus; the skin with pox is collected; the skin is fragmented; an aqueous solution of phenol is added, and the skin is soaked at a temperature of lower than about 12°C (for example, about 0-10°C, preferably about 2-8°C, more preferably about 3-6°C, more preferably about 4°C) for at least about 12 hours (for example, about 24-90 hours, preferably about 48-72 hours, more preferably about 70 or about 72 hours). The supernatant is obtained by centrifugation, and solution A is obtained by filtration. The phenol concentration in the phenol aqueous solution is about 1%-10%, preferably about 2%-5%, more preferably about 2% or about 3%.

In one aspect, in step (2), the solution A is adjusted to acidic (for example, about pH 4-6, more preferably about pH 4.5-5.5, more preferably about pH 5) with acid (for example, hydrochloric acid), and heated (for example, at about 90-100°C, preferably about 95°C for at least about 10 minutes, such as about 20-50 minutes, preferably about 30-40 minutes), optionally lowering the temperature (for example, to less than about 50°C, preferably less than about 30°C), followed by centrifugation to obtain the supernatant, and filtration to obtain solution B. The step (2) can be performed in a nitrogen environment.

In one aspect, in step (3), the solution B is adjusted to alkaline (e.g., about pH 8-10, more preferably about pH 8.5-9.5, more preferably about pH 9 or about pH 9. 2), and heated (for example, at about 90-100°C, preferably about 95°C for at least 10 minutes, such as about 30-50 minutes, preferably about 30-40 minutes), optionally lowering the temperature (for example, to less than about 50°C, preferably less than about 30°C), followed by filtration to obtain solution C. The step (3) can be performed in a nitrogen environment.

In one aspect, in step (4), the solution C is adjusted to acidic (for example, about pH 3-6, more preferably about pH 4-5, more preferably about pH 4.5) with an acid (for example, hydrochloric acid), and an adsorbent is added thereto (For example, activated carbon) for soaking (for example, under stirring for at least about 1 hour, preferably about 2-10 hours, more preferably about 4 hours), after which the solution is removed and the adsorbent containing the active ingredient is collected. Subsequently, the above-mentioned adsorbent is added to the eluent (for example, water), and the pH is adjusted to alkaline (for example, about pH 9-12, preferably about pH 10 or pH 11) with a base (for example, sodium hydroxide) to separate the active ingredient from the adsorbent (for example, stirring for at least about 1 hour, preferably 2-10 hours, more preferably 4 hours, followed by filtration, and then washing the adsorbent with water) to obtain solution D. The step (4) can be performed in a nitrogen environment.

In one aspect, in step (5), solution D is adjusted to weakly acidic (for example, about pH 5.5-6.6, preferably about pH 6) with acid (for example, hydrochloric acid) to obtain solution E. Preferably, the step (5) can be performed under aseptic conditions.

In one aspect, in step (6), the solution E is concentrated (for example, concentrated under reduced pressure, preferably concentrated by evaporation under reduced pressure, for example, at about 50°C to 70°C, preferably at about 54°C to 56°C), followed by filtration to obtain an extract containing the active ingredient. The step (6) can be performed in a nitrogen environment.

In one aspect, the patient is a mammal. In one aspect, the patient is a human, such as a middle-aged or elderly person, preferably an elderly person. The middle-aged person can be a person between 40 and 59 years old or a person between 45 and 59 years old. The elderly can be a person at or over 60 years old, at or over 65 years old, at or over 70 years old, at or over 75 years old, or at or over 80 years old.

Those skilled in the art understand that the extract of the present invention can also be obtained by inoculating other animal tissues with vaccinia virus. For example, in the present invention, an extract from an inflamed tissue inoculated with vaccinia virus can be used. The tissue may be derived from a mammalian tissue, and the mammal may comprise companion animal, laboratory animal, and livestock animal, such as rabbit, cow, horse, sheep, goat, monkey, mouse, and pig. The tissue may be skin.

### BRIEF DESCRIPTION OF THE DRAWINGS

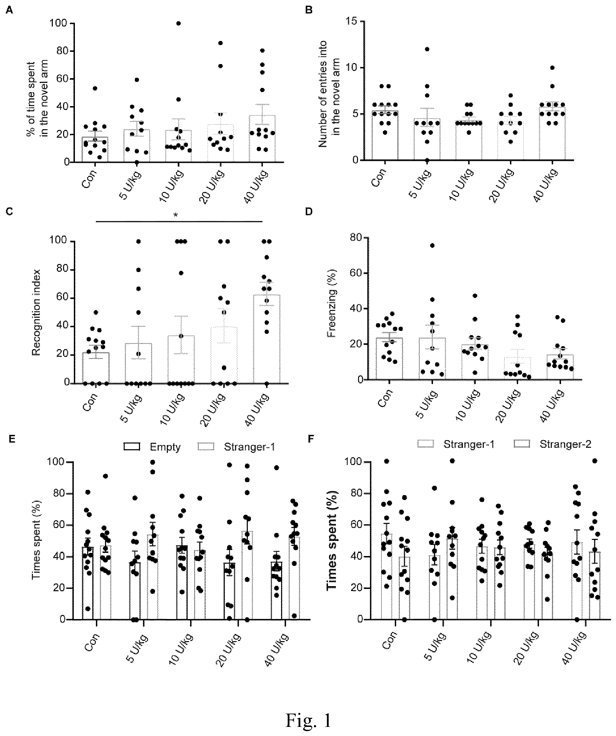

Figure 1 shows the effects of different doses of Lepalvir on short-term learning and memory, conditioned fear memory and social ability of APP/PS1 mouse. (A) % of time spent in the novel arm by the mouse in the Y-maze, (B) number of entries into the novel arm by the mouse in the Y-maze, (C) recognition index of mouse in the novel object recognition experiment, (D) The percentage of freezing time of mouse in the conditioned fear memory experiment, (E) % of time spent in Empty chamber and Stranger-1 chamber by the mouse in the social preference experiment, (F) % of time spent in Stranger-1 chamber and Stranger-2 chamber by the mouse in the social memory experiment. Data were statistically analyzed by one-way ANOVA with multiple comparison test (A-D) and two-way ANOVA (EF). p < 0.05 was considered statistically significant. Data are presented as mean ± standard error, and the black circles in the statistical graphs represent the number of mice, n = 11-13 per group (because male mice may fight when they were raised, causing local injuries to mice, and mice with poor behavior were eliminated). % of time spent in the novel arm: number of entries into the novel arm: recognition index; freezing: time spent.

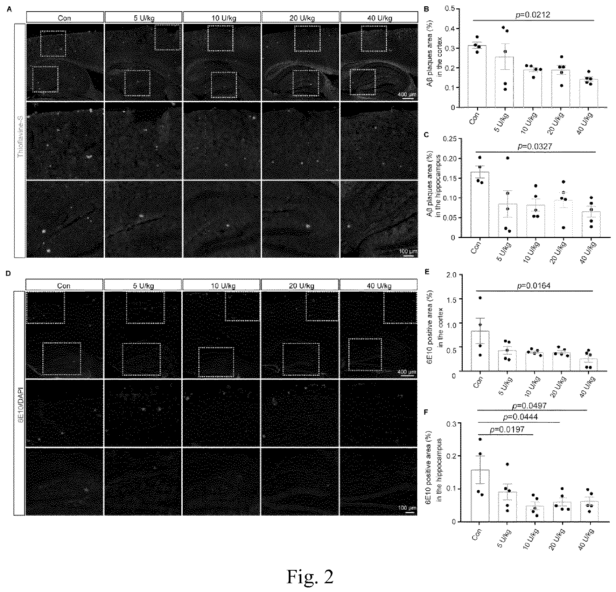

Figure 2 shows the effects of different doses of Lepalvir on Aβ deposition in APP/PS1 mouse. (A) Thioflavin-positive plaques in the cerebral cortex and hippocampus of mouse administered with different doses of Lepalvir were detected by immunofluorescence staining. (BC) Quantification of thioflavin-positive Aβ plaques in the cortex and hippocampus of mouse administered with different doses of Lepalvir. (D) 6E10-positive plaques in the cerebral cortex and hippocampus of mouse administered with different doses of Lepalvir were detected by immunofluorescence. (EF) Quantification of 6E10-positive plaques in the cortex and hippocampus of mouse administered with different doses of Lepalvir. Data were statistically analyzed by one-way ANOVA with multiple comparison test (BC, EF). p < 0.05 was considered statistically significant. Data are presented as mean ± standard error, and black circles in the statistical graphs represent the number of mice, n = 4-5 per group. Thioflavine-S; Aβ plaques area in the corte; Aβ plaques area in the hippocampus; 6E10 positive area in the cortex; 6E10 positive area in the hippocampus.

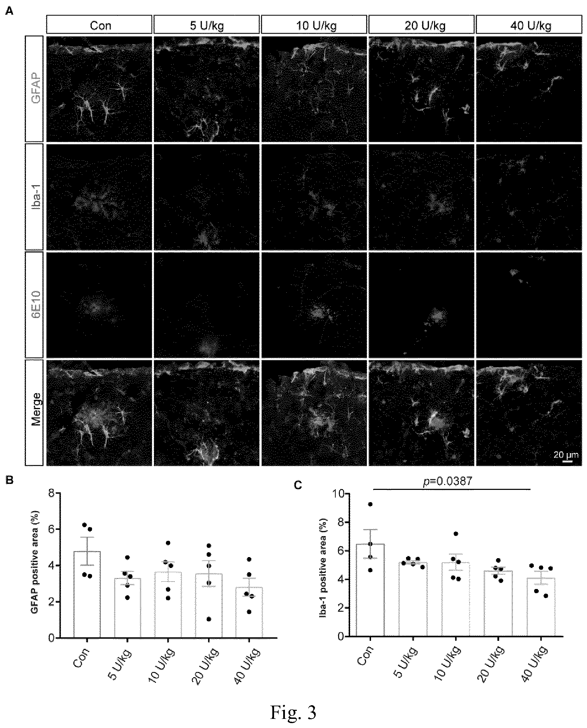

Figure 3 shows the effects of different doses of Lepalvir on the activation of glial cells around Aβ plaques in the cortex of APP/PS1 mouse. (A) GFAP, a marker of astrocytes, and Iba-1, a marker of microglia, in the cortex of mouse administered with different doses of Lepalvir were detected by immunofluorescence staining. (BC) The percentage of GFAP and Iba1 positive areas in the cortex of mouse administered with different doses of Lepalvir. Data were statistically analyzed by one-way ANOVA with multiple comparison test (BC). p < 0.05 was considered statistically significant. Data are presented as mean ± standard error, and the black circles in the statistical graphs represent the number of mice, n = 4-5 per group. GFAP positive area: Iba-1 positive area.

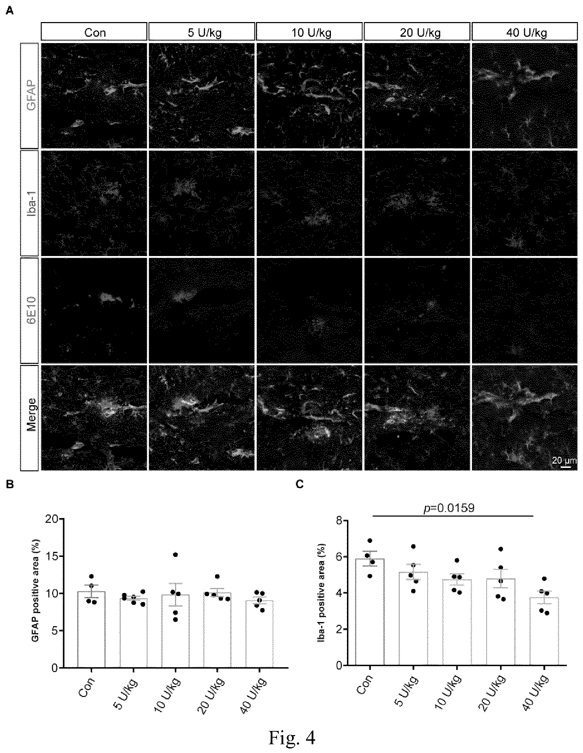

Figure 4 shows the effects of different doses of Lepalvir on the activation of glial cells around Aβ plaques in the hippocampus of APP/PS1 mouse. (A) GFAP, a marker of astrocytes, and Iba-1, a marker of microglia, in the hippocampus of mouse administered with different doses of Lepalvir were detected by immunofluorescence staining. (BC) The percentage of GFAP and Iba1 positive areas in the hippocampus of mouse administered with different doses of Lepalvir. Data were statistically analyzed by one-way ANOVA with multiple comparison test (BC). p < 0.05 was considered statistically significant. Data are presented as mean ± standard error, and the black circles in the statistical graphs represent the number of mice, n = 4-5 per group. GFAP positive area: Iba-1 positive area.

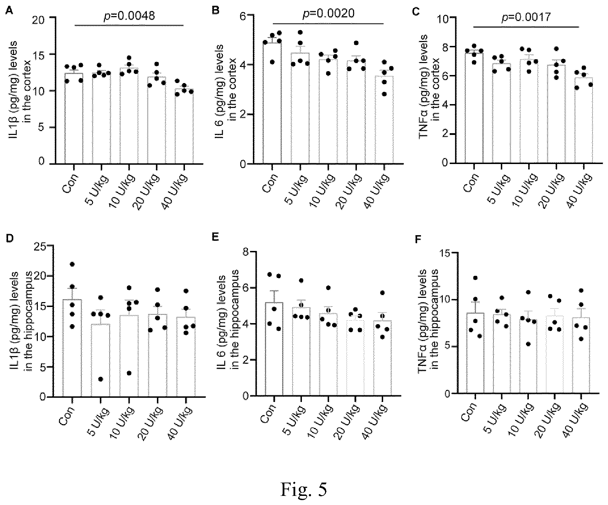

Figure 5 shows the effects of different doses of Lepalvir on inflammatory factors IL-1β, IL-6 and TNF- α in the cerebral cortex and hippocampus of APP/PS1 mouse. (AC) The expression of IL-1β, IL-6 and TNF-α in the cortex of mouse administered with different doses of Lepalvir were detected by ELISA. (DF) The expression of IL-1β, IL-6 and TNF-α in the hippocampus of mouse administered with Lepalvir were detected by ELISA. The data were statistically analyzed by one-way ANOVA with multiple comparison test (AF). p < 0.05 was considered statistically significant. The data are presented as mean ± standard error, and the black circles in the statistical graphs represent the number of mice, n = 5 per group. IL-1β levels in the cortex; IL-6 levels in the cortex; TNFα levels in the cortex; IL-1β levels in the hippocampus; IL-6 levels in the hippocampus; TNFα levels in the hippocampus.

### DETAILED DESCRIPTION

Unless otherwise specified, all scientific terms used herein have the same meaning as commonly understood by those of ordinary skill in the art. Exemplary methods and materials are described below, and equivalents can be used. All publications and other references mentioned herein are incorporated by reference in their entirety.

The following examples are provided to further illustrate the present invention. The following examples are not intended to limit the scope of the present invention for any reason.

### Examples

### Example 1 - Study on the neuroprotective effects of Lepalvir on the APP/PS1 mouse (model animal of Alzheimer's disease)

### I. Research methods

### 1. Administration of Lepalvir

APP/PS1 male mice of 5-month old on a C57BL/6 background were treated with 80 µL of saline and an equal volume of different doses of Lepalvir (5, 10, 20 and 40 U/kg) by intraperitoneal injection at the same time every day for 4 weeks. The total volume of intraperitoneal injection in each group was 80 µL, and the control group was given the same volume of saline. After each week of administration, 20-35 mice were randomly selected to measure their body weight, and the average body weight was used as an important basis for the next week's dosing.

### 2. Behavioral testing

### 2.1. Short-term learning and memory

### 2.1.1. Y-maze

The Y-maze device used in this experiment has an angle of 120 degrees between the three arms, which are interconnected. The three arms are of the same size, all of which are 29 cm × 8 cm × 15 cm in length, width and height. Different patterns are posted on the inside of the arms as visual markers for mice, and they are named starting arm, novel arm and other arms. The experimental process is divided into two parts, the adaptation phase and the test phase, with an interval of 1-2 hours. The adaptation is to separate the novel arm with a baffle, and the mice can only move freely in the starting arm and other arms. The adaptation time for each mouse is 5 minutes; and the test phase is to open the novel arm after 1-2 hours so that the mice can move freely in the three arms. The test time for each mouse is 5 minutes. During the experiment, the time and number of entries into the novel arm were recorded in real time using Topscan software. After each experiment, the experimental equipment was cleaned with 75% alcohol to remove the smell of mice.

### 2.1.2. Novel object recognition

Before testing, the mice were stroked every day to avoid stimulation during operation. In the first stage (familiarization phase), two identical objects (AB, make sure the objects had no smell and would not be pushed away) were placed into the device, 10 cm away from the two side walls, and the mice were placed from the same distance away from the objects into the device with their backs facing the objects. The camera and software were used to record the exploration time of the mice on each object (as long as the mouth or nose touched the object and approached the object within about 2-3 cm, the exploration of the object was counted). Within 5 minutes (many experiments have confirmed that after 2 minutes of familiarization, animals have a good preference for novel things, and of course the preference is more obvious after 3 minutes of familiarization), the number of times, period and distance of the animal's exploration of each object were measured. In the second stage (test phase), i.e., 1 hour after the completion of the first stage, a different object (AC or BC) replacing one of the two identical objects was introduced into the device. The mice were also placed from the same distance away from the objects into the device with their backs facing the objects, and kept for 5 minutes. The cognitive status of the mouse was detected by the number of times, period and distance of the mouse's exploration of new and old objects, that is, the number of times, period and distance of the mouse's activities around the new and old objects. If the mouse's cognitive ability is poor, there is no difference in the exploration of new and old objects; if the mouse's cognitive ability is normal, the exploration of new things is longer than that of old things. Calculation formula for the recognition index (RI) is: RI = new object/(new object + old object ) × 100%.

### 2.2. Conditioned fear memory

On the first day of this experiment, mice were placed in a box (with copper bars at the bottom of the box, powerable). After acclimation for 3 minutes, the mice were allowed to stay in the box for another 6 minutes. During this period, a single-frequency sound stimulus (1.0 KHZ, 70 db, 30 s) and an inescapable foot shock (0.8 mA, 2 s) were given every 2 minutes for a total of three times. The total time of sound- and electric shock-induced freezing behavior of the mice within 6 minutes was recorded, and then they were returned to the cage. After each experiment, the bottom of the box was wiped with 75% alcohol. After 24 hours, the mice with established conditioned fear were placed in the original box and immediately given the same intensity of sound stimulation (1.0 KHZ, 70 db, 30 s). The sound-induced freezing behavior of the mice was recorded within 3 minutes. Freezing behavior is defined as no other movement behavior except breathing. The percentage of freezing time induced by environment and sound was recorded.

### 2.3. Social behavior

### 2.3.1. Social preference stage

Before the experiment, mice were placed in the behavior test room to adapt for 0.5 h; the rectangular box to be tested was divided into three areas: Empty, Center, and Stranger 1; mice of the same sex were placed in the metal cage in the Stranger 1 area, and the metal cage at the other side of the box was kept empty; the test mice were put into the box in the middle (Center) so that the test mice could move freely in the three boxes for 5 min; relevant parameters were immediately photographed and recorded: Regarding the duration of entry into each box, when the head and four paws of the mouse entered a box, it was considered to be in that box.

### 2.3.2. Social memory stage

In the social memory stage of the experiment, a stranger mouse of the same species (Stranger 2) was placed in another metal cage, and then the time spent by the test mouse in the Stranger 1 and Stranger 2 areas was recorded for 5 min.

### 3. Brain tissue collection and slice preparation

### 3.1. Sample collection

After the behavioral test of mice was completed, samples were collected in time. Blood was collected through the eyeball after anesthesia. Physiological saline was perfused from the left ventricle of the heart until the liver turned earthy yellow, and the physiological saline was replaced with 4% paraformaldehyde solution, which was perfused for 5 min. The brain was fixed in 4% paraformaldehyde at 4°C overnight. The brain tissue containing the hippocampus and cortex was cut and dehydrated with sucrose gradient.

### 3.2. Frozen section preparation

The brain tissue embedded in OCT resin was sliced into serial sagittal sections with a thickness of 20 µm using a Leica Microtome Cryostat, which were collected into PBS for immunofluorescence staining and Aβ pathological histological analysis.

### 4. Immunofluorescence staining

The sections were blocked with 10% goat serum PBS solution at room temperature for 1 h. The blocking solution was discarded, and mouse 6E10 antibody, chicken glial fibrillary acidic protein (GFAP) antibody and rabbit ionized calcium binding adapter molecule 1, Iba1) antibody were added for incubation at 4°C overnight. The primary antibody was washed off the next day, with PBS wash three times, 5 min each time. Secondary antibodies of corresponding species were added (goat anti-chicken IgG-488; donkey anti-rabbit IgG-488; and donkey anti-mouse IgG-647) for incubation at room temperature in the dark for 1 h. The secondary antibody was washed off, with PBS wash three times, 5 min each time. The slides were sealed with an anti-fluorescence quencher, and photographed under a fluorescence microscope.

### 5. Thioflavin S staining

Thioflavin S can mark Aβ core plaques on brain slices. 1% thioflavin solution was added to the slices. The slices were incubated at room temperature for 5 min, rinsed with running water for 1 min, separated with 70% alcohol for 30s, sealed with an anti-fluorescence quencher, and photographed under a fluorescence microscope.

### 6. Image analysis

Immunohistochemically stained tissue sections were photographed using a Leica microscope. The percentage of GFAP, Iba1, 6E10, and thioflavin positive areas was counted by Image J software using grayscale threshold analysis method. There were 4-5 mice in each group, and 3 sections were selected for each mouse. The average value was taken as the statistical result of the group.

### 7. Enzyme-linked immunosorbent assay (ELISA)

### 7.1. Sample collection

After the mice were anesthetized, they were decapitated. The skin of the neck and head was peeled off to expose the skull, and the skull was cut open along the sagittal suture. The brain tissue was removed after the skull was separated. The cortical tissue and hippocampal tissue were placed in labeled centrifuge tubes, quickly frozen in liquid nitrogen, and transferred to a -80°C refrigerator for storage.

### 7.2. Sample preparation

10-20 mg of prefrontal cortex and hippocampus tissues were taken on the ice box, weighed in a 1.5 mL centrifuge tube, and then RIPA lysis buffer was added at a mass-volume ratio of 1:10. The remaining tissue was put back into the original tube, and frozen quickly with liquid nitrogen. At the same time, two steel balls were added to the centrifuge tube, which was placed into the homogenizer. The centrifuge tube was balanced, homogenized and lysed, and then shaken on ice for 30 minutes. After removal of the steel balls, the centrifuge tube was centrifuged at 12000 rpm, 4°C, 15 minutes, and the supernatant was pipetted sufficiently to a new 1.5 mL centrifuge tube. Protein concentration was quantified by the protein quantification method.

### 7.3. ELISA

The sample was diluted to an appropriate concentration by adding diluent, and the diluted sample was added to the wells of the plate reader for testing three proinflammatory indicators: interleukin-1β (IL-1β), IL-6, and tumor necrosis factor-α (TNF-α). Each sample was added into the wells at least in duplicates, and both the standard and test sample were 45 µL per well. Then, 50 µL of biotinylated antibody working solution was added to the wells of test sample and standard. The plate with reaction wells was sealed with sealing paper and incubated at room temperature for 2 h using a micro-oscillator at a frequency of 300 rpm. The enzyme-bound substrate was prepared 30 min in advance and placed at room temperature in the dark. The liquid was discarded from the well, and then the well was patted dry on an absorbent paper, washed 5 times. The washing procedure was as follows: the reaction solution in the well was aspirated; and the washing solution was added to the well plate, which was allowed to stand for 2 min with slight shaking. 100 µL of enzyme-binding substrate working solution was added to each well except the blank well, and the plate was incubated at room temperature for 1 h using a micro-oscillator at a frequency of 300 rpm. The liquid was discarded from the well, and then the well was patted dry on an absorbent paper, washed 5 times. 100 µL of chromogenic substrate working solution was added to each well except the blank well, and the plate was placed at room temperature in the dark for 15 min. 100 µL of stop solution was added to each well except the blank well to stop the reaction, and the experimental results were determined within 20 min. The absorbance of the standard wells and test wells was detected at a wavelength of 450 nm. A standard curve was plotted based on the standard wells and known concentrations, and then the actual concentration of the test wells was calculated from the standard curve. Finally, the target protein mass per mg of brain tissue was obtained based on the protein concentration.

### II. Research results

### 1. Effects of different doses of Lepalvir on memory behavior of APP/PS1 mouse

Y-maze was used to evaluate the short-term memory of each group of mice. The results showed that compared with the control group, the time spent in the novel arm by the APP/PS1 mouse increased with increasing doses of Lepalvir, but there was no significant difference; the number of entries into the novel arm did not increase significantly after the administration of Lepalvir (5-40 U/kg) (Figure 1A-B). The short-term memory of each group of mice was evaluated by novel object recognition. The results showed that the index of APP/PS1 mouse recognizing novel objects increased with increasing doses of Lepalvir (5-40 U/kg), wherein the index of mouse recognizing novel objects in the 40 U/kg group increased significantly (Figure 1C)*. Conditioned fear memory was used to evaluate the fear memory of each group of mice. The results showed that compared with the control group, the percentage of freezing time of APP/PS1 mouse did not decrease significantly with increasing doses of Lepalvir (5-40 U/kg) (Figure 1D). The three-box experiment was used to evaluate the social ability of each group of mice. The results showed that in the social preference stage, the mice in the 5-10 U / kg group had no obvious preference for mice of the same sex, while the 20-40 U / kg group showed social preference, although there was no significant difference (Figure 1E ) (normal WT mouse showed a greater preference for mouse of the same sex); in the social memory stage, the groups at all doses did not show a significant preference for strange mouse, indicating that social ability was not significantly improved (Figure 1F). These results are also shown in Table 1.

**Table 1 Effects of different doses of Lepalvir on short-term learning and memory, conditioned fear memory and social ability of APP/PS1 mouse**

| Group | Num ber | Y-maze test | | Novel object recognition - novel object recognition index | Conditioned fear memory-percentage of freezing time | Social skills | | | |
|---|---|---|---|---|---|---|---|---|---|
| | | Percentage of time spent in the novel arm | Number of entries into the novel arm | | | Social Preference | | Social Memory | |
| | | | | | | Percentage of time spent in Empty chamber | Percentage of time spent in Stranger1 chamber | Percentage of time spent in Stranger1 chamber | Percentage of time spent in Stranger2 chamber |
| Control group (Con) | 13 | 18.85 ±12.93 | 5.46 ± 1.45 | 22.27 ± 16.98 | 23.99 ± 9.39 | 46.41 ± 19.16 | 47.42 ± 16.26 | 54.20 ± 23.01 | 39.92 ± 22.25 |
| 5 U/kg | 11 | 24.18 ± 17.72 | 4.64 ± 3.26 | 28.86 ± 38.23 | 23.96 ± 22.37 | 36.69 ± 22.57 | 54.29 ± 24.54 | 40.94 ± 21.02 | 51.12 ± 22.47 |
| 10 U/kg | 12 | 23.82 ± 26.07 | 4.42 ± 0.90 | 34.26 ± 45.80 | 20.40 ± 11.16 | 47.33 ± 17.26 | 44.35 ± 16.84 | 46.26 ± 14.94 | 45.64 ± 15.58 |
| 20 U/kg | 11 | 28.13 ± 25.70 | 4.36 ± 1.43 | 40.61 ± 40.04 | 13.16 ± 13.31 | 36.45 ± 27.21 | 56.48 ± 27.94 | 47.8 ± 19.32 | 41.45 ± 13.16 |
| 40 U/kg | 12 | 34.50 ± 25.15 | 5.83 ± 1.70 | 63.07 ± 28.50 * | 14.60 ± 10.04 | 37.21 ± 21.15 | 52.87 ± 19.49 | 48.92 ± 25.98 | 42.98 ± 25.90 |

| | | | | | | | | | |
|---|---|---|---|---|---|---|---|---|---|
| **p*<0.05 *vs.* Control group. | | | | | | | | | |

### 2. Effects of different doses of Lepalvir on the alleviation of Aβ plaque deposition in APP/PS1 mouse

Thioflavin staining was used to evaluate the Aβ deposition in each group of mice. The results showed that compared with the control group, the Aβ plaques in the hippocampus and cortex of mouse were reduced with increasing doses of Lepalvir (5-40 U/kg), wherein there was a significant difference in the reduction of Aβ plaques in the 40 U/kg group (Figure 2A - C). 6E10 staining showed that the Aβ plaques in the hippocampus of mouse in the 10, 20 and 40 U /kg groups were significantly reduced, and for the cortical Aβ plaques, the reduction in the 40 U/kg group was significantly different (Figure 2D-F). Thioflavin binds to mature Aβ plaques, while 6E10 binds to amino acid residues 1-16 of Aβ protein. Therefore, Thioflavin mainly detects larger fibrous Aβ plaques, while 6E10 can detect not only larger Aβ plaques, but also scattered small Aβ plaques, and 6E10 staining is thus more sensitive than Thioflavin staining. The results are also shown in Table 2.

**Table 2 Effects of different doses of Lepalvir on Aβ deposition in APP/PS1 mouse**

| Group | Numb er | Thioflavin S stain ring | | 6E10 staining | |
|---|---|---|---|---|---|
| | | Percentage of Aβ plaque deposition in the cortex | Percentage of Aβ plaque deposition in the hippocampus | Percentage of Aβ plaque deposition in the cortex | Percentage of Aβ plaque deposition in the hippocampus |
| Control group (Con) | 4 | 0.34 ± 0.08 | 0.17 ± 0.03 | 0.84 ± 0.53 | 0.16 ± 0.09 |
| 5 U/kg | 5 | 0.21 ± 0.11 | 0.08 ± 0.08 | 0.43 ± 0.18 | 0.09 ± 0.06 |
| 10 U/kg | 5 | 0.17 ± 0.04 | 0.08 ± 0.03 | 0.40 ± 0.05 | 0.05 ± 0.03 * |
| 20 U/kg | 5 | 0.20 ± 0.11 | 0.09 ± 0.04 | 0.39 ± 0.07 | 0.06 ± 0.03 * |
| 40 U/kg | 5 | 0.20 ± 0.06 * | 0.07 ± 0.03 * | 0.26 ± 0.17 * | 0.06 ± 0.03 * |

| | | | | | |
|---|---|---|---|---|---|
| ** p < 0.05 vs.* Control group. | | | | | |

### 3. Effects of different doses of Lepalvir on the activation of glial cells around Aβ plaques in the cortex of APP/PS1 mouse

The activation of astrocytes (GFAP staining) and microglia (Iba1 staining) around Aβ plaques in the cortex of APP/PS1 mouse was evaluated by co-labeling with 6E10, GFAP and Iba1. The results showed that compared with the control group, after administration of Lepalvir (5-40U/kg), the activation of astrocytes in the cerebral cortex and hippocampus of mouse had a decreasing trend without significant difference, while after administration of Lepalvir (40 U/kg), the activation of microglia in the cerebral cortex and hippocampus of mouse was significantly reduced (Figures 3 and 4). The results are also shown in Tables 3 and 4.

**Table 3 and Table 4 Effects of different doses of Lepalvir on the activation of glial cells around Aβ plaques in the cortex and hippocampus of APP/PS1 mouse**

| Group | Number | Cortex | | Seahorse | |
|---|---|---|---|---|---|
| | | % area of astrocytes | % area of microglial cells | % area of astrocytes | % area of microglial cells |
| Control group (Con) | 4 | 4.79 ± 1.54 | 6.49 ± 2.00 | 10.28 ± 1.67 | 5.90 ± 0.81 |
| 5 U/kg | 5 | 3.31 ± 0.82 | 5.19 ± 0.25 | 9.28 ±0.67 | 5.17 ±0.94 |
| 10 U/kg | 5 | 3.66 ±1.22 | 5.20 ± 1.28 | 9.84± 3.39 | 4.76 ± 0.71 |
| 20 U/kg | 5 | 3.56 ± 1.60 | 4.60 ± 0.55 | 10.12 ± 1.24 | 4.80± 1.15 |
| 40 U/kg | 5 | 2.81 ±1.13 | 4.11± 1.02 * | 9.07±1.03 | 3.76 ± 0.79 * |

| | | | | | |
|---|---|---|---|---|---|
| ** p <* 0.05 *vs.* control group. | | | | | |

### 4. Effects of different doses of Lepalvir on the reduction of inflammatory factors in the cortex and hippocampus of APP/PS1 mouse

The expression of inflammatory factors IL-1β, IL-6 and TNF-α in the cerebral cortex of each group of mice was evaluated by enzyme-linked immunosorbent assay. The results showed that compared with the control group, the 40 U/kg Lepalvir dose group significantly reduced the expression of inflammatory factors IL-1β, IL-6 and TNF-α in the cerebral cortex of APP/PS1 mouse (Figure 5A-C). Compared with the control group, different dose groups of Lepalvir had no significant improvement in the expression of inflammatory factors in the hippocampus of APP/PS1 mouse (Figure 5D-F). The results are also shown in Table 5.

### III. Conclusion

Lepalvir, e.g., at 40 U/kg, significantly alleviated short-term cognitive dysfunction in AD model mouse. Meanwhile, Lepalvir, e.g., at 40 U/kg, reduced Aβ load and microglial inflammatory response. The neuroprotective effects of Lepalvir on short-term cognitive dysfunction in AD model mouse may be achieved by reducing Aβ load and microglial inflammatory response.

**Table 5 Effects of different doses of Lepalvir on IL-1β, IL-6 and TNF-α inflammatory factors in the cerebral cortex and hippocampus of APP/PS1 mouse**

| Group | Number | Cortex | | | Hippocampus | | |
|---|---|---|---|---|---|---|---|
| | | IL-1β content | IL-6 content | TNF-α content | IL-1β content | IL-6 content | TNF-α content |
| Control group (Con) | 5 | 12.62 ± 0.98 | 4.88 ± 0.46 | 7.56 ± 0.42 | 16.16 ± 4.02 | 5.19 ± **1.43** | 8.60 ± 2.56 |
| 5 U/kg | 5 | 12.49 ± 0.59 | 4.47 ± 0.58 | 6.86 ± 0.46 | 12.07 ± 5.23 | 4.92 ± 0.88 | 8.45 ± 1.16 |
| 10 U/kg | 5 | 13.13 ± 0.91 | 4.22 ± 0.35 | 7.15 ± 0.65 | 13.55 ± 5.50 | 4.58 ± 0.86 | 7.91 ± 1.96 |
| 20 U/kg | 5 | 11.92 ± 1.17 | 4.17 ± 0.42 | 6.74 ± 0.75 | 13.72 ± 2.79 | 4.22 ± 0.53 | 8.31 ± 1.70 |
| 40 U/kg | 5 | 10.30 ± 0.74 ** | 3.55 ± 0.50 ** | 5.88 ± 0.58 ** | 13.23 ± 2.84 | 4.20 ± 0.95 | 8.11 ± 2.08 |

| | | | | | | | |
|---|---|---|---|---|---|---|---|
| ** *p* < 0.01 *vs*. control group. | | | | | | | |

## Claims

1. Use of extract from rabbit skin inflamed by vaccinia virus in the preparation of a medicament for preventing or treating Alzheimer's disease in a patient.

2. The use according to claim 1, wherein the medicament is used to protect neurological function of the brain or alleviate damage to neurological function of the brain in a patient suffering from Alzheimer's disease.

3. The use according to claim 1, wherein the prevention or treatment is achieved by alleviating or reducing β-amyloid plaque deposition, inflammatory response of glial cells, or the expression or level of inflammatory factors in the brain.

4. Use of extract from rabbit skin inflamed by vaccinia virus in the preparation of a medicament for alleviating or reducing β-amyloid plaque deposition in the brain of a patient suffering from Alzheimer's disease.

5. Use of extract from rabbit skin inflamed by vaccinia virus in the preparation of a medicament for alleviating or reducing inflammatory response of glial cells in the brain of a patient suffering from Alzheimer's disease.

6. The use according to claim 3 or 5, wherein the glial cell is selected from microglia or astrocyte.

7. Use of extract from rabbit skin inflamed by vaccinia virus in the preparation of a medicament for alleviating or reducing the expression or level of inflammatory factors in the brain of a patient suffering from Alzheimer's disease.

8. The use according to claim 3 or 7, wherein the inflammatory factor is selected from IL-1β, IL-6 or TNF-α.

9. Use of extract from rabbit skin inflamed by vaccinia virus in the preparation of a medicament for improving, enhancing or restoring cognitive functions or cognitive abilities, preferably including perception, logical thinking, memory, language, learning, emotion control, social skills or attention, in a patient suffering from Alzheimer's disease.

10. The use according to any one of claims 1 to 9, wherein the extract from rabbit skin inflamed by vaccinia virus is Lepalvir.

11. The use according to any one of claims 1 to 10, wherein the extract from rabbit skin inflamed by vaccinia virus is formulated into an oral preparation or an injection, preferably an intramuscular injection or an intravenous injection.

12. The use according to any one of claims 1 to 11, wherein the patient is a human, preferably an elderly person.

13. The use according to any one of claims 1 to 12, wherein the extract from rabbit skin inflamed by vaccinia virus is administered to the patient in an amount of 0.05 U/kg to 50 U/kg, preferably 0.1 U/kg to 10 U/kg, more preferably 0.5 U/kg to 5 U/kg.

14. The use according to any one of claims 1 to 13, wherein the medicament comprises 3 U to 3000 U, preferably 6 U to 600 U, more preferably 30 U to 300 U of the extract from rabbit skin inflamed by vaccinia virus.
